# EUROPEAN PATENT SPECIFICATION

(11) **EP 3 794 340 B1**
(45) Date of publication and mention of the grant of the patent: **28.10.2026**
(21) Application number: 19802478.8
(22) Date of filing: 15.05.2019
(51) Int. Cl.: G01N 27/414, C12Q 1/00

(54) **BIOSENSOR WITH POROUS WICKING LAYER**
BIOSENSOR MIT PORÖSER DOCHTSCHICHT
BIOCAPTEUR AVEC COUCHE POREUSE À EFFET DE MÈCHE

(30) Priority: 15.05.2018 AU 2018901675
(43) Date of publication of application: 24.03.2021
(73) Proprietor: The University of Newcastle, Callaghan, New South Wales 2308 (AU)
(72) Inventor: DASTOOR, Paul, Gosforth, New South Wales 2320 (AU)
(74) Representative: Turner, Craig Robert
(86) International application number: PCT/AU2019/050458
(87) International publication number: WO 2019/218011

(56) References cited:
- WO-A1-2018/000012
- WO-A1-2018/000012
- JP-A- S61 274 682
- US-A1- 2017 343 508
- US-A1- 2017 343 508
- XUE H ET AL: "A highly stable biosensor for phenols prepared by immobilizing polyphenol oxidase into polyaniline-polyacrylonitrile composite matrix", TALANTA, vol. 57, no. 2, 16 May 2002 (2002-05-16), NL, pages 289 - 295, XP055874194, ISSN: 0039-9140, DOI: 10.1016/S0039-9140(02)00028-0
- ELKINGTON, D. ET AL.: "Detection of saliva-range glucose concentrations using organic thin-film transistors", APPLIED PHYSICS LETTERS, vol. 105, 2014, pages 043303 - 1-043303-4, XP012188148, DOI: 10.1063/1.4892012
- FRYCZKOWSKA, BEATA ET AL.: "Preparation and properties of composite PAN/PANI membranes", INTERNATIONAL JOURNAL OF POLYMER SCIENCE, vol. 2017, 2017, XP055653575
- JOONWON BAE, KIM IL T, HUR JAEHYUN: "Field-Effect Transistors Based on Organic and Carbon-Based Materials for Chemical and Biological Sensors", CURRENT ORGANIC CHEMISTRY, vol. 19, no. 12, 2015, pages 1176 - 1190, XP055760833
- TORSI, LUISA ET AL.: "Organic thin-film transistors as plastic analytical sensors", ANALYTICAL CHEMISTRY, 2005, XP055653578

## Description

### Field of the invention

The present invention relates to organic thin film sensors and the preparation and use thereof in sensing applications, and in particular in glucose sensing applications.

### Background of the invention

The development of organic thin film transistors (OTFTs) sensors has grown rapidly in recent years motivated primarily by the unique physical properties of polymer devices, including their flexibility and ability to be fabricated using low-cost, solution-based techniques. Work on developing OTFTs for new and existing applications has focussed on two main areas. First, there have been systematic improvements in the materials and fabrication processes which have led to an improvement in the conventional performance parameters of organic devices making them comparable to their inorganic counterparts. Second, improvements in film morphology of the organic semiconducting layer have been made with the goal of eliminating electron and/or hole traps and enhancing free carrier transport in the polymer semiconducting materials. Progress has also been made in developing high capacitance organic dielectric layers and large improvements in OTFT performance have been reported. The inherent compatibility of organic materials with biological molecules makes OTFTs suitable for use in bio sensing applications.

The present inventors have successfully fabricated an OTFT device that is capable of detecting analyte levels across a broad range of concentrations and which is straightforward and relatively cheap to manufacture. The device may open the way for a commercially viable glucose sensor that allows blood glucose concentration to be estimated by detecting the level of glucose in saliva as opposed to blood. Such a device may obviate the need for diabetic patients to obtain a blood sample when determining their blood glucose level. The device may also open the way for sensing of other analytes in a similar fashion.

US 2017/0343508 A1 discloses a transistor device comprising: a gate electrode comprising a sulfonated tetrafluoroethylene-based fluoropolymer-copolymer and glucose oxidase, the gate electrode being disposed above, and in direct contact with, a dielectric layer, the dielectric layer being disposed above, and in direct contact with, a semiconducting layer, the semiconducting layer being disposed above and in between a source electrode and a drain electrode.

Reference to any prior art in the specification is not an acknowledgment or suggestion that this prior art forms part of the common general knowledge in any jurisdiction or that this prior art could reasonably be expected to be understood, regarded as relevant, and/or combined with other pieces of prior art by a skilled person in the art.

### Summary of the invention

In a first aspect of the invention there is provided an organic thin film transistor based sensor for detecting the presence of an analyte in an aqueous liquid sample as defined in claim 1.

In an embodiment, the sensor further includes an ohmic conductor in contact with the polymer layer configured to apply a gate voltage to the polymer gating layer.

In an embodiment, the porous layer has a thickness of from about 50 nm up to about 500 µm. Preferably, the thickness is up to 200 µm. More preferably, the thickness is up to 100 µm. Even more preferably, the thickness is up to 80 µm. Most preferably, the thickness is up to 60 µm. Alternatively, or additionally, the thickness is from 500 nm. More preferably the thickness is from 1 µm. Even more preferably, the thickness is from 20 µm. Most preferably, the thickness is from 40 µm. In a preferred form, the thickness is about 50 µm.

In an embodiment, the porous polymer wicking layer is formed from a material such that the contact angle of the liquid, preferably biofluid, more preferably diluted saliva, or undiluted saliva or unprocessed saliva ('neat saliva') on the first surface of the porous polymer wicking layer is 60° or less. Preferably, the contact angle is 50° or less. More preferably, the contact angle is 45° or less. Even more preferably, the contact angle is 40° or less. Most preferably, the contact angle is 38° or less.

In an embodiment, the porous polymer wicking layer includes a plurality of pores having a pore size of from 50 nm up to 2000 nm. Preferably, the pore size is up to 1600nm. More preferably, the pore size is up to 1200 nm. Most preferably, the pore size is up to 1000 nm. Alternatively, or additionally, the pore size is from 80 nm. More preferably the pore size is from 100 nm. Most preferably, the pore size is from 150 nm.

In an embodiment the porous polymer wicking layer has a void ratio of from about 30% up to about 95%. Preferably the void ratio is up to about 90%. Alternatively, or additionally, the void ratio is from about 35%. Preferably, the void ratio is from about 40%. More preferably, the void ratio is from about 45%. Most preferably, the void ratio is from about 50%. The porous polymer wicking layer may be a layer of a homopolymer, a copolymer, or a polymer blend.

In certain forms, the porous polymer wicking layer includes, consists of, or consists essentially of a porous polymer, the enzyme, and optionally one or more additives selected from the group consisting of an elastomer, a stabiliser, or a surfactant.

It is preferred that the porous polymer wicking layer is formed from a polymer that has a glass transition temperature of at least 50 °C. More preferably, the glass transition temperature is at least 70 °C. Even more preferably, the glass transition temperature is at least 80 °C. Still more preferably, the glass transition temperature is at least 90 °C. Most preferably, the glass transition temperature is about 95 °C.

It is preferred that the porous polymer wicking layer is formed from a polymer that is soluble in an organic solvent. Preferably, the polymer is soluble in a polar aprotic solvent, such as dimethyl sulfoxide.

It preferred that the porous polymer wicking layer is formed from a polymer that is formed from a one or more repeating monomer units, wherein the one or more repeating monomer units do not include a halide atom. Preferably, the one or more repeating monomer units consist of C, N, O, and H atoms. More preferably, the one or more repeating monomer units consist of C, N, and H atoms.

In an embodiment, the porous polymer wicking layer is a porous polyacrylonitrile (PAN) layer.

In one or more embodiments, the porous polymer wicking layer is directly layered on a surface of the polymer gating layer.

In one or more embodiments, the polymer gating layer is directly layered on a surface of the organic semiconducting layer.

In one or more embodiments, the sensor also comprises a substrate layer. Preferably, at least the source electrode and drain electrode are disposed on the substrate. In at least one form, the source electrode, drain electrode and organic semiconductor are each in contact with the substrate. The substrate may be glass, or any other suitable substrate known to those skilled in the art, for example paper or a low-cost plastic, such as polyethylene terephthalate (PET).

In a preferred form of the invention, the analyte is glucose, and the enzyme is glucose oxidase.

In a second aspect of the invention, there is provided a method of forming an organic thin film transistor based sensor for detecting the presence of an analyte in a liquid sample, the method including:
providing a layered structure including at least an organic semiconducting layer configured to be connected to a source electrode and a drain electrode, and a polymer gating layer; and
disposing a porous polymer wicking layer on a surface of the polymer gating layer;
wherein the porous polymer wicking layer includes, or is treated to include an enzyme for facilitating the generation of a charge carrier from an analyte.

In one embodiment, the step of disposing a porous polymer wicking layer on the surface of the polymer gating layer includes forming the porous polymer wicking layer on the surface of the polymer gating layer via a phase inversion method.

In one or more embodiments, the phase inversion method includes:
forming a wet film of a polymer solution on the surface of a substrate, the polymer solution including at least a polymer dissolved in a solvent; and
applying a non-solvent to the wet film, the non-solvent being miscible with the solvent, to form a porous polymer membrane and to dissolve the solvent from pores of the porous polymer membrane.

In one form the substrate is a surface of the polymer gating layer. In an alternative form, the substrate is an inert layer that is separate from the organic thin film transistor based sensor, and the method further includes removing the porous wicking layer from the substrate prior to disposing the porous wicking layer on the surface of the polymer gating layer.

Further aspects of the present invention and further embodiments of the aspects described in the preceding paragraphs will become apparent from the following description, given by way of example and with reference to the accompanying drawings.

### Brief description of the drawings

**Figure 1****:** Schematic illustrating the structure of a device in accordance with one embodiment of the invention.
**Figure 2****:** Schematic illustrating the structure of a device in accordance with one embodiment of the invention.
**Figure 3****:** Scanning electron micrographs (SEM) of poly(acrylonitrile) (PAN) porous membranes. The scale bar in (a) is 100 µm and the scale bar in (b) is 1 µm.
**Figure 4****:** Photograph depicting the contact angle of water on a glucose sensor (a) with poly(acrylonitrile) (PAN) and (b) without PAN.
**Figure 5****:** (a) XPS survey scan of pristine Nafion film. (b) XPS region scans of pristine PAN membrane (solid line), Nafion/PAN bilayer (dashed line) and Nafion/PAN bilayer exposed to analyte solution (dotted line).
**Figure 6****:** Schematic depicting sensor device architecture incorporating porous poly(acrylonitrile) membranes, (a) layer 1, electrodes, ITO; (b) layer 2, polymer semiconductor, P3HT; (c) layer 3, gate electrode, Nafion; (d) layer 4, porous membrane, PAN; (e) layer 5, enzyme, GOX. Each substrate pictured contains two sensors.
**Figure 7****:** (a) Photograph of sensors placed on inkjet printer platen following printing of GOX layer, the sensors on the left contain a PAN porous membrane and the sensors on the right do not contain a PAN porous membrane. (b) Sensor response to a 10 mM glucose solution added at t = 180 s to a device containing a PAN membrane (solid line) and a device with no PAN membrane (dashed line).

### Detailed description of the embodiments

In a first aspect of the invention there is provided an organic thin film transistor based sensor for detecting the presence of an analyte in an aqueous liquid sample as defined in claim 1.

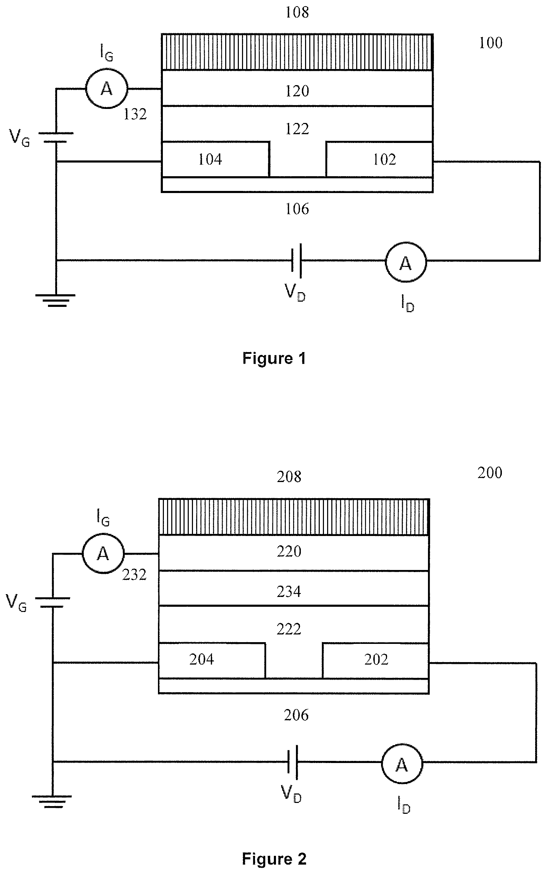

An exemplary organic thin film transistor based sensor 100 in accordance with one embodiment of the invention is illustrated in **Figure 1****,** which provides a conceptual representation of the structure of the sensor 100. Sensor 100 includes a drain electrode 102 and source electrode 104 disposed on the surface of a substrate 106. The sensor includes a three-layered film structure that includes: a porous wicking layer 108, a polymer gating layer 120, and an organic semiconducting layer 122. The organic semiconducting layer 122 covers a portion of the drain and source electrodes, with the organic semiconducting layer 108 in contact with, and extending between the drain electrode 102 and the source electrode 104. The polymer gating layer 120 is disposed on a surface of the organic semiconducting layer 122. An ohmic conductor 132 is in contact with polymer gating layer 120 to enable a gate voltage to be applied to the polymer gating layer 120. A porous polymer wicking layer 108 is disposed on the surface of the polymer gating layer 120. The surface of the porous polymer wicking layer 108 is exposed to receive a fluid sample. An enzyme is disposed within and/or on the surface of the porous wicking layer 108. The porous polymer wicking layer 100 is not located between gate and drain electrodes. The porous polymer wicking layer 100 is not configured to enable an ohmic conductor to connect to the porous polymer wicking layer.

The inventors have found that incorporation of a porous polymer wicking layer including an enzyme into an organic thin film transistor is advantageous. This is for two primary reasons. Firstly, on depositing a liquid sample onto the surface of the porous polymer wicking layer, the porous polymer wicking layer exhibits a wicking effect which improves incorporation of a liquid sample into the device, this wicking effect has the potential to ensure delivery of a standard volume of liquid sample to within the device. Secondly, the wicking effect results in excellent wetting of the internal porous structure of the porous polymer wicking layer which results in a high degree of exposure of an analyte within the liquid sample to the enzyme retained within the porous polymer wicking layer. This is beneficial as it results in efficient generation of charge carriers within the device, and consequently the rapid detection of the analyte if present within the liquid sample. Suitable polymers for the porous polymer wicking layer 108 include, but are not limited to: polyacrylonitrile, polysulfone, polyethersulfone, polystyrene, polybutadiene, polyisoprene, polyimides , polyamides, and fluoropolymers. **The** porous polymer wicking layer is formed from a material that is not the same as the material that the polymer gating layer is formed from. Where the polymer gating layer is formed from Nafion, the porous polymer wicking layer is not formed from Nafion. Preferably the porous polymer wicking layer is not formed from Nafion. Ideally the porous polymer wicking layer 108 is formed from a material that has a low contact angle with the liquid of the liquid sample to ensure that the liquid sample is rapidly taken into the porous polymer wicking layer 108 and that there is good wetting of pore surfaces of the porous polymer wicking layer 108.

Preferably the porous polymer wicking layer takes the form of a layer that enables the rapid formation of a droplet, preferably an aqueous droplet, more preferably a biological fluid droplet, more preferably a saliva droplet, such as a neat saliva droplet or a processed or diluted saliva droplet, on the porous polymer wicking layer that has a low contact angle with the porous polymer wicking layer.

Preferably the porous polymer wicking layer is formed from a layer having a high degree of wettability by an aqueous droplet, more preferably a biological fluid droplet, more preferably a saliva droplet, more preferably a neat saliva droplet or a processed or diluted saliva droplet. Notwithstanding the above, it is preferred that the porous polymer wicking layer 108 is a porous poly(acrylonitrile) (PAN) layer. PAN has physical properties that make it particularly useful as the porous wicking layer 108. PAN is thermally stable with a glass transition temperature (T_{g}) of ~95 °C and melting temperature (Tₘ) > 300 °C. Given this, PAN exhibits good thermal stability, which is desirable in a sensor as this means that during typical use, the T_{g} is unlikely to be exceeded. If the T_{g} is exceeded, the porous structure may collapse. PAN also has high strength, a high modulus of elasticity, and a low density. Furthermore, from a fabrication point of view, PAN is soluble in dimethyl sulfoxide (DMSO) and can be used to prepare porous polymer membranes via the phase inversion technique-immersion precipitation.

In a preferred embodiment, the porous polymer wicking layer has a sufficiently low surface energy to enable a body fluid, preferably saliva, preferably undiluted or otherwise unprocessed saliva, or processd saliva to rapidly wet and penetrate the porous polymer wicking layer.

As discussed above, the porous polymer wicking layer 108 includes an enzyme. The enzyme may be disposed on a surface of the porous polymer wicking layer 108 and/or disposed throughout the porous polymer wicking layer 108, such that the enzyme is exposed on pore surfaces within the porous polymer wicking layer 108.

The enzyme is selected to facilitate generation of charge carriers when an analyte contacts the device. The charge carriers are typically electrons, anions, or cations (e.g. hydrogen ions/protons). The generation of the charge carriers may be further facilitated by the presence of an electric field. As will be described, these generated charge carriers can then contribute to electric current through the device. It will be recognised that a range of enzymes could be used for any one particular analyte. Further given the diversity of enzymes available, the device, by following the disclosure herein can be adapted or developed for detection of a range of analytes.

A particularly preferred class of enzyme is an oxidoreductase. An oxidoreductase for use in the device may act on any one of the following donor groups: the CH-OH group of donors (alcohol oxidoreductases), the aldehyde or oxo group of donors, the CH-CH group of donors (CH-CH oxidoreductases), the CH-NH₂ group of donors (amino acid oxidoreductases, monoamine oxidase), CH-NH group of donors, NADH or NADPH, other nitrogenous compounds as donors, a sulfur group of donors, a heme group of donors, diphenols and related substances as donors, peroxide as an acceptor (peroxidases), hydrogen as donors, single donors with incorporation of molecular oxygen (oxygenases), paired donors with incorporation of molecular oxygen, superoxide radicals as acceptors, CH or CH₂ groups, iron-sulfur proteins as donors, reduced flavodoxin as a donor, phosphorus or arsenic in donors, X-H and Y-H to form an X-Y bond, or oxidoreductases that oxidize metal ions.

The polymer gating layer 120 is disposed between the porous polymer wicking layer 108 and the organic semiconducting layer 122. The function of polymer gating layer 120 is to facilitate transport of charge carriers generated in the porous polymer wicking layer 108 from an analyte on application of a gate voltage. A range of different polymers may be used to form the polymer gating layer 120 depending on the nature of the analyte, enzyme, and/or charge carrier. In a preferred form, the charge carriers are protons (such as hydrogen ions) and the polymer gating layer 120 is proton conductive. Preferably the polymer layer has a conductivity to protons that is greater than a conductivity to protons of the organic semiconductor layer 122. The conductivity may be due to permeability of the polymer gating layer 120 to charge carriers, such as where conduction occurs via migration of the charge carriers; alternatively, conduction may occur via another mechanism, such as the Grotthuss mechanism. Where a charge carrier is a proton, or is an electron, the polymer gating layer may be proton conductive or electron conductive.

In preferred forms of the invention, the polymer gating layer 120 comprises, consists, or consists essentially of a sulfonated tetrafluoroethylene-based fluoropolymer-copolymer, for example a copolymer comprising a tetrafluoroethylene backbone and perfluoroalkyl ether groups terminated with sulfonate groups. More preferably, the sulfonated tetrafluoroethylene-based fluoropolymer-copolymer is a copolymer of tetrafluoroethylene and perfluoro-3,6-dioxa-4-methyl-7-octene-sulfonic acid. An example of a suitable polymer is tetrafluoroethylene-perfluoro-3,6-dioxa-4-methyl-7-octenesulfonic acid copolymer (commonly referred to as Nafion).

In preferred forms of the invention the polymer gating layer has a thickness in the range of from about 50 nm to about 5 µm. It is preferred that the thickness is from about 200 to about 800 nm.

The organic semiconducting layer 122 is configured to enable flow of electrical current between the source electrode and the drain electrode as a result of the generation of these charge carriers.

The organic semiconducting layer 122 includes one or more organic compounds. Any organic compound having semiconducting properties is suitable for use. However, it is preferred that the one or more organic compounds are selected from the group consisting of: polyacetylenes, porphyrins, phthalocyanins, fullerenes, polyparaphenylenes, polyphenylenevinylenes, polyfluorenes, polythiophenes, polypyrroles, polypyridines, polycarbazoles, polypyridinevinylenes, polyarylvinylenes, poly (p-phenylmethylvinylenes), including derivatives and co-polymers thereof, and further including combinations thereof. More preferably, the one or more organic compounds are selected from the group consisting of: poly(9,9-dioctylfluorene-2,7-diyl-co-bis-N,N-(4- butylphenyl)-bis-N,N-phenyl- 1 ,4-phenylenediamine), poly(9,9-dioctylfluorene-2,7-diyl-co-benzothiadiazole), poly(3-hexylthiophene), (6,6)-phenyl-C₆₁-butyric acid methyl ester, poly(2-methoxy-5-(2'-ethyl-hexyloxy)-1,4-phenylene vinylene), and combinations thereof. Most preferably, the semiconducting layer includes, consists of, or consists essentially of P3HT.

In preferred forms of the invention, the organic semiconducting layer has a layer thickness of from 20 nm up to 500 nm. Preferably, the layer thickness is up to 350 nm. More preferably, the layer thickness is up to 200 nm. Most preferably, the layer thickness is up to 150 nm. Alternatively, or additionally, the layer thickness is from 40 nm. More preferably the layer thickness is from 60 nm. Most preferably, the layer thickness is from 70 nm.

The use of the sensor 100 will now be described below in relation to a preferred embodiment in which the sensor 100 is for detecting the presence of glucose in a saliva sample.

In use, a gate voltage V_{G} and a drain voltage V_{D} are applied to the sensor 100 (the voltages being with respect to the source electrode 104, as shown in Figure 1), and a liquid sample comprising glucose, for example a bodily fluid such as saliva, is contacted with the porous polymer wicking layer 108. The liquid sample is wicked into the internal porous structure of the porous polymer wicking layer, where glucose in the liquid sample comes into contact with glucose oxidase enzyme (GOX) that is present within the porous polymer wicking layer. The glucose is degraded via an enzymatic reaction with GOX thereby producing H₂O₂. The gate voltage V_{G} and drain voltage V_{D} provide a sufficiently strong electric field to liberate H⁺ from H₂O₂, but not strong enough to cause electrolysis of water, as electrolysis of water may lead to a decrease in the signal-to-noise ratio of the sensor. Typically, the gate voltage and drain voltage applied are between about 0 V and -2 V, e.g. about -1 V.

The H⁺ ions are conducted from the porous polymer wicking layer 108 though the polymer gating layer 120 (e.g. Nafion) to the organic semiconducting layer. This results in doping of the semiconductor, and consequentially, current between the drain and the source electrodes. Without wishing to be bound by theory, the inventors are of the view that the gate potential controls the doping and de-doping of the semiconducting compound(s) via ion migration from the site of ion generation to the active channel in the organic semiconductor. Thus, the increase in H⁺ ions results in an increase in drain current, such that a relationship is established between the amount of glucose present in the sample and the magnitude of the drain current. The drain current is then measured which provides an indication of the presence and concentration of glucose within the saliva sample.

The gate voltage V_{G} and drain voltage V_{D} provide a sufficiently strong electric field to liberate H⁺ from H₂O₂, but not enough to cause electrolysis of water, as electrolysis of water may lead to a decrease in the signal-to-noise ratio of the sensor. In at least one embodiment, the gate voltage and drain voltage applied are between about 0 V and -2 V, e.g. about -1 V.

In an alternative form of the invention, as illustrated in Figure 2, the sensor 200 is similar to sensor 100 of Figure 1, but further comprises a dielectric layer 234 between and in contact with polymer gating layer 220 and organic semiconductor layer 222.

The dielectric layer 234 comprises, consists of, or consists essentially of an organic dielectric material. Preferably, the organic dielectric material has a conductivity to protons that is greater than the conductivity of organic semiconductor layer 222. Preferably the organic dielectric material is a hygroscopic insulator, such as for example polyvinyl phenols. More specifically, the dielectric layer may comprise, consist of, or consist essentially of, poly(4-vinylphenol).

Alternative dielectric materials that may be used in the devices will be readily apparent to those skilled in the art. Non-limiting examples include polyimide and poly(methyl methacrylate (PMMA). In alternative embodiments the dielectric layer may comprise a doped dielectric material, for example lithium perchlorate doped poly(4-vinylpyridine).

### Example

### Fabrication of the device

Pre-patterned ITO-on-glass substrates (15 Ω □⁻¹ ITO, Xin Yan Technology) were used as the substrate and electrodes of the devices. Poly-3-hexylthiophene (P3HT) (MW ~20 000) was dissolved in CHCl₃ (Sigma-Aldrich) at various concentrations and sonicated for ~1 hour or until the material was entirely dissolved. Poly-4-vinylphenol (PVP) (Sigma-Aldrich) was dissolved in ethanol (Sigma-Aldrich) at a concentration of 80 mg mL⁻¹ and sonicated for ~1 hour or until the material was entirely dissolved. Nafion solution (5 % by weight in lower aliphatic alcohols and water, Sigma-Aldrich) was used as received.

The pre-patterned ITO-on-glass substrates were cleaned with methanol and purified water. P3HT solution in CHCl₃ was spin-coated onto the substrates at 2000 rpm for 60 seconds. P3HT solutions of 5 mg mL⁻¹, 10 mg mL⁻¹, 15 mg mL⁻¹, 20 mg mL⁻¹, and 40 mg mL⁻¹ were prepared, with average thicknesses of films spun from these concentrations of P3HT were 22 nm, 36 nm, 74 nm, 108 nm and 390 nm respectively. The P3HT layer was patterned and then left to dry for 15 minutes at 40 °C. Nafion solution was first spin-coated at 500 rpm for 120 seconds.

While the devices exemplified herein do not include a dielectric layer, a dielectric layer may be included. A preferred dielelectric layer is a PVP layer. For devices including a PVP layer, PVP solution can be spun on top of the P3HT layer at 2000 rpm for 60 seconds to form a film of thickness ~400 nm, which can then patterned and dried. For PVP-containing devices, Nafion can then be drop-cast above the source-drain channel area and connected to the ITO gate pad of the substrate before being dried for approximately 30 minutes.

Porous polymer membranes can be fabricated using the phase inversion (immersion precipitation) technique. Firstly, a viscous solution of polyacrylonitrile (PAN) polymer in DMSO solvent is coated on a flat, rigid substrate, such as glass, to form a uniform 'wet' film. The 'wet' film can be coated by either spin coating, drop-casting or with the use of a printing technique such as slot-die coating.The 'wet' film is not a solid, dry film, it is a composite film, comprising a polymer network with liquid solvent molecules existing in the voids between polymer chains. The 'wet' film upon the rigid substrate is then submerged in water (the non-solvent), the porous polymer membrane forming as the DMSO solvent diffuses out of the polymer network into the surrounding water bath and the water diffuses into the polymer network. It is this solvent exchange with non-solvent that induces precipitation of the polymer. The selection of solvent and non-solvent is highly dependent on the chosen polymer, the solvent needs to be capable of dissolving the polymer whereas the non-solvent is one in which the polymer is insoluble. The solvent and non-solvent combination should be miscible, otherwise the solvent will not diffuse into the non-solvent and the porous polymer membrane will not form, instead remaining as a 'wet' solvent-containing film. The fabrication is a two-step process, it is during the second step that the 'wet' film transforms into a solid film. Once the solid film has been formed, it is processed into individual layers of the desired size and thickness and applied to the device as a porous wicking layer.

To incorporate GOX into the wicking layer, the GOX is cast on top of the wicking layer. The porosity of the wicking layer promotes the dispersion of the GOX throughout the wicking layer.

### Testing of the device

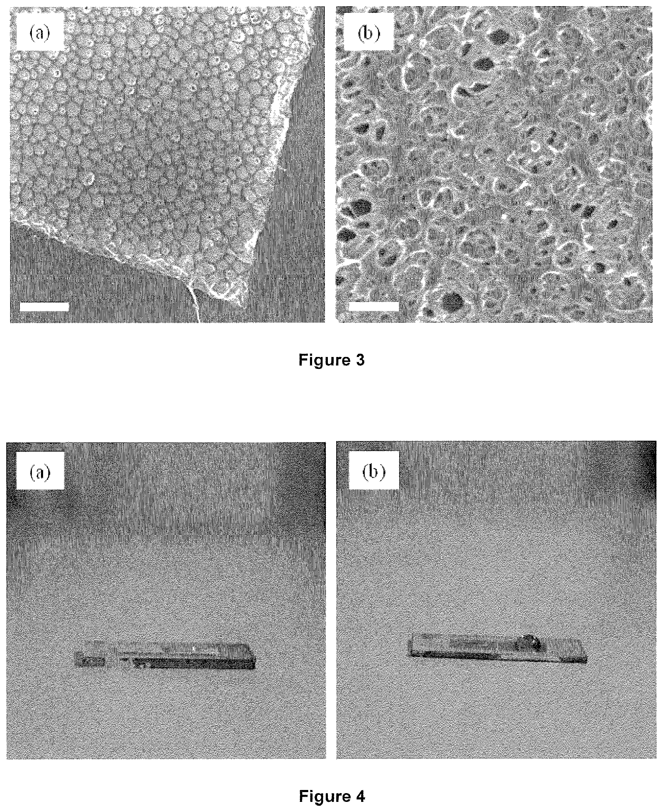

The porosity of PAN membranes prepared via the phase inversion technique were investigated using scanning electron microscopy (SEM) (see **Figure 3**) on a Zeiss ZP FESEM operating at an accelerating voltage of 2 kV. DMSO was used as the solvent and water as the non-solvent in the membrane preparation process. SEM revealed PAN membrane pore size to be in the 200 - 800 nm size range.

To investigate the potential for wicking of PAN membranes, the contact angle of water on PAN compared to water on Nafion was measured. **Figure 4** presents photographs of water droplets on (a) PAN and (b) Nafion from which the contact angle was extracted. The contact angle of water on PAN was measured to be ~35 °, this increased to ~70 ° on Nafion. The decreased contact angle of water on PAN indicates that PAN is a more hydrophilic surface than Nafion. This reduced contact angle on PAN is beneficial for a wicking effect, an excellent result for initial trials of this material.

X-ray photoelectron spectroscopy (XPS) was performed to probe any potential redistribution of Nafion throughout the PAN porous membranes following analyte addition. XPS spectra were collected by illuminating the samples with a non-monochromatic X-ray source (Omnivac) using Al Kα (1486.6 eV) radiation, and the photoemission collected by an SES2002 analyser (Scienta). The F 1s peak was utilised as a marker for Nafion as Nafion contains fluorine atoms attached to the carbon atoms of the polymer backbone whereas PAN only contains the elements carbon, nitrogen and hydrogen. Structures for (a) PAN and (b) Nafion are provided below:

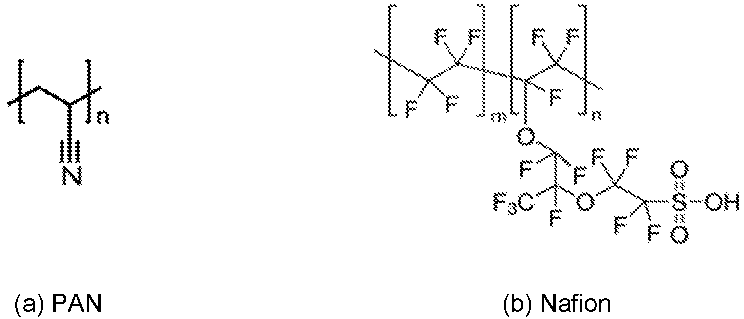

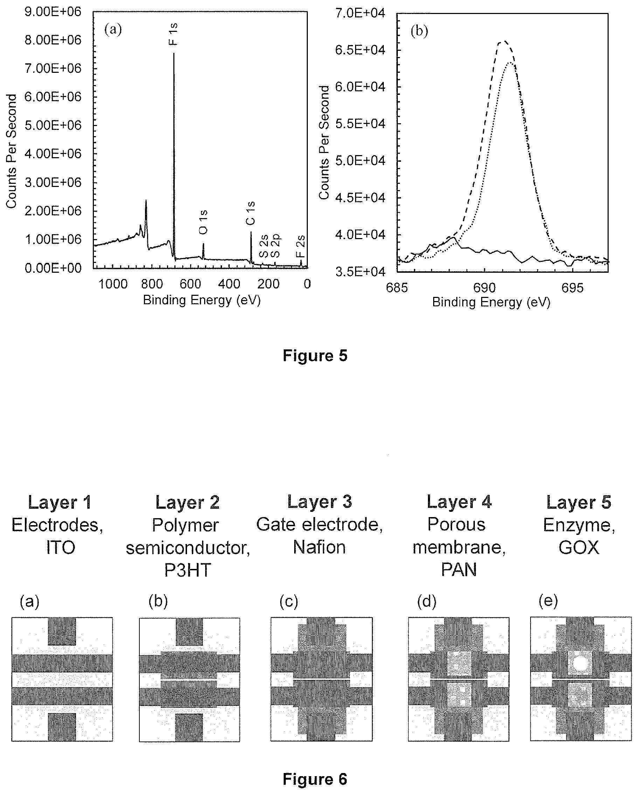

An XPS survey scan (**Figure 5**(a)) of a pristine Nafion film identified the F 1s peak at a binding energy of 691 eV. XPS region scans were then performed of a pristine PAN membrane, a Nafion/PAN bilayer and a Nafion/PAN bilayer following exposure to analyte solution. The pristine PAN membrane had no F 1s peak (**Figure 5**(b)). The Nafion/PAN bilayer XPS spectrum contains a peak for F 1s, the Nafion/PAN bilayer following exposure to analyte solution XPS spectrum also contains a peak for F 1s, and this peak is quite invariant compared to the Nafion/PAN bilayer indicating that the analyte solution does not cause solvation and redistribution of the underlying Nafion film. This is a positive result for the biosensors indicating that the Nafion film is quite robust.

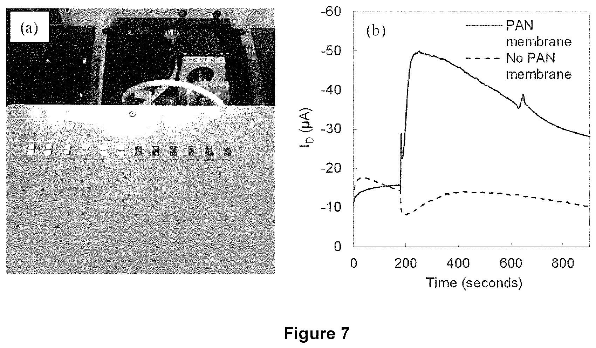

Glucose sensors were fabricated with the architecture depicted in **Figure 6****.** Sensors with PAN and without PAN were fabricated and the GOX layer was inkjet printed with a Dimatix DMP 2831 inkjet printer with a 10 pL nozzle printing head (**Figure 7**).

The sensors were exposed to glucose analyte solutions and the difference in response of sensors with and without PAN porous membranes was analysed. **Figure 7**(b) presents representative plots of a sensor with PAN and a sensor without PAN following exposure to a 10 mM glucose solution. Here we observe an improvement in the drain current (ID) response time as well as the maximum current when we utilise porous PAN membranes. The rate of ID increase changes from 0.04 µA/s without PAN to 0.93 µA/s with PAN, and we observe a more than three-fold improvement in total ID for the PAN-containing sensor. The superior response time suggests that glucose is oxidised at a faster rate and/or the hydrogen peroxide is able to travel to the Nafion layer more quickly.

It will be understood that the invention disclosed and defined in this specification extends to all alternative combinations of two or more of the individual features mentioned or evident from the text or drawings. All of these different combinations constitute various alternative aspects of the invention. The scope of the present invention is defined by the appended claims.

## Claims

1. An organic thin film transistor based sensor (100, 200) for detecting the presence of an analyte in a aqueous liquid sample, the sensor including:
a porous polymer wicking layer (108, 208) having a first surface and a second surface, wherein the first surface is configured to receive the liquid sample;
an enzyme disposed on or within the porous polymer wicking layer, the enzyme for facilitating the generation of a charge carrier from the analyte;
a polymer gating layer (120, 220) in contact with the second surface of the porous wicking layer, and configured to be connected to an ohmic conductor (132, 232) for applying a gate voltage to the polymer gating layer, the polymer gating layer being conductive to the charge carrier;
a drain electrode (102, 202) and a source electrode (104, 204); and
an organic semiconducting layer (122, 222) connected to source electrode and the drain electrode,
wherein the porous polymer wicking layer is a different material than the polymer gating layer.

2. The sensor of claim 1, wherein the porous polymer wicking layer has a thickness of from 50 nm up to about 500 µm.

3. The sensor of claim 1 or 2, wherein the porous polymer wicking layer is formed from a material such that the contact angle of the liquid on the first surface of the porous polymer wicking layer is 60° or less, preferably the contact angle is 50° or less, more preferably the contact angle is 40° or less.

4. The sensor of any one of claims 1 to 3, wherein the pore size of the porous polymer wicking layer is from 50 nm to 2000 nm, preferably the pore size is from 100 nm to 1000 nm.

5. The sensor of any one of claims 1 to 4, wherein the porous polymer wicking layer has a void ratio of from about 30% up to about 95%.

6. The sensor of any one of claims 1 to 5, wherein the porous polymer wicking layer is formed from a polymer that has a glass transition temperature of at least 80 °C, preferably the glass transition temperature is at least 90°C.

7. The sensor of any one of claims 1 to 6, wherein the porous polymer wicking layer is formed from a polymer that is soluble in dimethyl sulfoxide.

8. The sensor of any one of claims 1 to 7, wherein the porous polymer wicking layer is formed from a polymer that is formed from a one or more repeating monomer units, wherein the one or more repeating monomer units do not include a halide atom.

9. The sensor of claim 8, wherein the one or more repeating monomer units consist of C, N, and H atoms.

10. The sensor of any one of the preceding claims, wherein the porous polymer wicking layer is a porous polyacrylonitrile (PAN) layer.

11. The sensor of any one of claims 1 to 10, wherein the enzyme is within the porous polymer wicking layer.

12. The sensor of any one of claims 1 to 11, wherein the analyte is glucose and the enzyme is glucose oxidase.

13. The sensor of any one of claims 1 to 10, wherein the polymer gating layer comprises a sulfonated tetrafluoroethylene-based fluoropolymer-copolymer, preferably tetrafluoroethylene-perfluoro-3,6-dioxa-4-methyl-7-octenesulfonic acid copolymer.

14. The sensor of any one of claims 1 to 13, wherein the organic semiconducting layer includes one or more organic polymers selected from the group consisting of polyacetylenes, porphyrins, phthalocyanins, fullerenes, polyparaphenylenes, polyphenylenevinylenes, polyfluorenes, polythiophenes, polypyrroles, polypyridines, polycarbazoles, polypyridinevinylenes, polyarylvinylenes, poly(p-phenylmethylvinylenes), preferably poly-3-hexylthiophene.

15. A method of forming an organic thin film transistor based sensor (100, 200) according to any one of claims 1 to 14 for detecting the presence of an analyte in an aqueous liquid sample, the method including:
providing a layered structure including at least an organic semiconducting layer (122, 222) connected to a source electrode (104, 204) and a drain electrode (102, 202), and a polymer gating layer (120, 220); and
disposing a porous polymer wicking layer (108, 208) on a surface of the polymer gating layer;
wherein the porous polymer wicking layer includes, or is treated to include an enzyme for facilitating the generation of a charge carrier from an analyte.

16. The method of claim 15, wherein the step of disposing a porous polymer wicking layer on the surface of the polymer gating layer includes forming the porous polymer wicking layer on the surface of the polymer gating layer via a phase inversion method.

17. The method of claim 16, wherein the phase inversion method includes:
forming a wet film of a polymer solution on the surface of a substrate, the polymer solution including at least a polymer dissolved in a solvent; and
applying a non-solvent to the wet film, the non-solvent being miscible with the solvent, to form a porous polymer membrane and to dissolve the solvent from pores of the porous polymer membrane.

## Patentansprüche

1. Sensor (100, 200) auf Basis eines organischen Dünnfilmtransistors zum Nachweisen des Vorhandenseins eines Analyten in einer wässrigen Flüssigkeitsprobe, wobei der Sensor Folgendes enthält:
eine poröse Polymer-Dochtschicht (108, 208) mit einer ersten Oberfläche und einer zweiten Oberfläche,
wobei die erste Oberfläche dazu konfiguriert ist, die Flüssigkeitsprobe aufzunehmen;
ein Enzym, das auf oder innerhalb der porösen Polymer-Dochtschicht angeordnet ist, wobei das Enzym die Erzeugung eines Ladungsträgers aus dem Analyten erleichtert;
eine Polymer-Gate-Schicht (120, 220), die in Kontakt mit der zweiten Oberfläche der porösen Dochtschicht steht und dazu konfiguriert ist, mit einem ohmschen Leiter (132, 232) zum Anlegen einer Gate-Spannung an die Polymer-Gate-Schicht verbunden zu werden, wobei die Polymer-Gate-Schicht für den Ladungsträger leitend ist;
eine Drain-Elektrode (102, 202) und eine Source-Elektrode (104, 204); und
eine organische halbleitende Schicht (122, 222), die mit der Source-Elektrode und der Drain-Elektrode verbunden ist,
wobei die poröse Polymer-Dochtschicht ein anderes Material als die Polymer-Gate-Schicht ist.

2. Sensor nach Anspruch 1, wobei die poröse Polymer-Dochtschicht eine Dicke von 50 nm bis etwa 500 µm aufweist.

3. Sensor nach Anspruch 1 oder 2, wobei die poröse Polymer-Dochtschicht aus einem solchen Material ausgebildet ist, sodass der Kontaktwinkel der Flüssigkeit auf der ersten Oberfläche der porösen Polymer-Dochtschicht 60° oder weniger beträgt, bevorzugt der Kontaktwinkel 50° oder weniger beträgt, besonders bevorzugt der Kontaktwinkel 40° oder weniger beträgt.

4. Sensor nach einem der Ansprüche 1 bis 3, wobei die Porengröße der porösen Polymer-Dochtschicht von 50 nm bis 2000 nm beträgt, bevorzugt die Porengröße von 100 nm bis 1000 nm beträgt.

5. Sensor nach einem der Ansprüche 1 bis 4, wobei die poröse Polymer-Dochtschicht ein Hohlraumverhältnis von etwa 30 % bis etwa 95 % aufweist.

6. Sensor nach einem der Ansprüche 1 bis 5, wobei die poröse Polymer-Dochtschicht aus einem Polymer ausgebildet ist, das eine Glasübergangstemperatur von mindestens 80 °C aufweist, wobei bevorzugt die Glasübergangstemperatur mindestens 90 °C beträgt.

7. Sensor nach einem der Ansprüche 1 bis 6, wobei die poröse Polymer-Dochtschicht aus einem Polymer ausgebildet ist, das in Dimethylsulfoxid löslich ist.

8. Sensor nach einem der Ansprüche 1 bis 7, wobei die poröse Polymer-Dochtschicht aus einem Polymer ausgebildet ist, das aus einer oder mehreren sich wiederholenden Monomereinheiten ausgebildet ist, wobei die eine oder mehreren sich wiederholenden Monomereinheiten kein Halogenidatom enthalten.

9. Sensor nach Anspruch 8, wobei die eine oder mehreren sich wiederholenden Monomereinheiten aus C-, N- und H-Atomen bestehen.

10. Sensor nach einem der vorhergehenden Ansprüche, wobei die poröse Polymer-Dochtschicht eine poröse Polyacrylnitril(PAN)-Schicht ist.

11. Sensor nach einem der Ansprüche 1 bis 10, wobei sich das Enzym innerhalb der porösen Polymer-Dochtschicht befindet.

12. Sensor nach einem der Ansprüche 1 bis 11, wobei der Analyt Glucose ist und das Enzym Glucoseoxidase ist.

13. Sensor nach einem der Ansprüche 1 bis 10, wobei die Polymer-Gate-Schicht ein sulfoniertes Fluorpolymer-Copolymer auf Tetrafluorethylenbasis, bevorzugt Tetrafluorethylen-perfluor-3,6-dioxa-4-methyl-7-octensulfonsäure-Copolymer, umfasst.

14. Sensor nach einem der Ansprüche 1 bis 13, wobei die organische halbleitende Schicht ein oder mehrere organische Polymere enthält, die aus der Gruppe ausgewählt sind, die besteht aus Polyacetylenen, Porphyrinen, Phthalocyaninen, Fullerenen, Polyparaphenylenen, Polyphenylenvinylenen, Polyfluorenen, Polythiophenen, Polypyrrolen, Polypyridinen, Polycarbazolen, Polypyridinvinylenen, Polyarylvinylenen, Poly(p-phenylmethylvinylenen), bevorzugt Poly-3-hexylthiophen.

15. Verfahren zum Ausbilden eines Sensors (100, 200) auf Basis eines organischen Dünnfilmtransistors gemäß einem der Ansprüche 1 bis 14 zum Nachweisen des Vorhandenseins eines Analyten in einer wässrigen Flüssigkeitsprobe, wobei das Verfahren Folgendes enthält:
Bereitstellen einer Schichtstruktur, die mindestens eine organische halbleitende Schicht (122, 222), die mit einer Source-Elektrode (104, 204) und einer Drain-Elektrode (102, 202) verbunden ist, und eine Polymer-Gate-Schicht (120, 220) enthält; und
Anordnen einer porösen Polymer-Dochtschicht (108, 208) auf einer Oberfläche der Polymer-Gate-Schicht;
wobei die poröse Polymer-Dochtschicht ein Enzym enthält oder behandelt ist, sodass es ein Enzym enthält, um die Erzeugung eines Ladungsträgers aus einem Analyten zu erleichtern.

16. Verfahren nach Anspruch 15, wobei der Schritt des Anordnens einer porösen Polymer-Dochtschicht auf der Oberfläche der Polymer-Gate-Schicht Ausbilden der porösen Polymer-Dochtschicht auf der Oberfläche der Polymer-Gate-Schicht über ein Phasenumkehrverfahren enthält.

17. Verfahren nach Anspruch 16, wobei das Phasenumkehrverfahren Folgendes enthält:
Ausbilden eines Nassfilms einer Polymerlösung auf der Oberfläche eines Substrats, wobei die Polymerlösung mindestens ein in einem Lösungsmittel gelöstes Polymer enthält; und
Aufbringen eines Nichtlösungsmittels auf den Nassfilm, wobei das Nichtlösungsmittel mit dem Lösungsmittel mischbar ist, um eine poröse Polymermembran auszubilden und das Lösungsmittel aus Poren der porösen Polymermembran zu lösen.

## Revendications

1. Capteur à base de transistor en couches minces organique (100, 200) destiné à détecter la présence d'un analyte dans un échantillon liquide aqueux, le capteur comprenant :
une couche polymère poreuse à effet de mèche (108, 208) présentant une première surface et une seconde surface,
dans lequel la première surface est conçue pour recevoir l'échantillon liquide ;
une enzyme disposée sur la couche polymère poreuse à effet de mèche ou au sein de celle-ci, l'enzyme étant destinée à faciliter la génération d'une porteuse de charge à partir de l'analyte ;
une couche de grille polymère (120, 220) en contact avec la seconde surface de la couche poreuse à effet de mèche, et conçue pour être connectée à un conducteur ohmique (132, 232) pour appliquer une tension de grille à la couche de grille polymère, la couche de grille polymère étant conductrice à la porteuse de charge ;
une électrode de drain (102, 202) et une électrode de source (104, 204) ; et
une couche semi-conductrice organique (122, 222) connectée à l'électrode de source et à l'électrode de drain,
dans lequel la couche polymère poreuse à effet de mèche est d'un matériau différent de la couche de grille polymère.

2. Capteur de la revendication 1, dans lequel la couche polymère poreuse à effet de mèche présente une épaisseur de 50 nm à environ 500 µm.

3. Capteur de la revendication 1 ou 2, dans lequel la couche polymère poreuse à effet de mèche est formée à partir d'un matériau de sorte que l'angle de contact du liquide sur la première surface de la couche polymère poreuse à effet de mèche est de 60° ou moins, de préférence l'angle de contact est de 50° ou moins, idéalement l'angle de contact est de 40° ou moins.

4. Capteur de l'une quelconque des revendications 1 à 3, dans lequel la taille des pores de la couche polymère poreuse à effet de mèche est de 50 nm à 2 000 nm, de préférence la taille des pores est de 100 nm à 1 000 nm.

5. Capteur de l'une quelconque des revendications 1 à 4, dans lequel la couche polymère poreuse à effet de mèche présente un rapport de vide d'environ 30 % à environ 95 %.

6. Capteur de l'une quelconque des revendications 1 à 5, dans lequel la couche polymère poreuse à effet de mèche est formée à partir d'un polymère qui présente une température de transition vitreuse d'au moins 80 °C, de préférence la température de transition vitreuse est d'au moins 90 °C.

7. Capteur de l'une quelconque des revendications 1 à 6, dans lequel la couche polymère poreuse à effet de mèche est formée à partir d'un polymère qui est soluble dans le diméthylsulfoxyde.

8. Capteur de l'une quelconque des revendications 1 à 7, dans lequel la couche polymère poreuse à effet de mèche est formée à partir d'un polymère qui est formé à partir d'une ou de plusieurs unités monomères répétitives, dans lequel les une ou plusieurs unités monomères répétitives ne comprennent pas d'atome d'halogénure.

9. Capteur de la revendication 8, dans lequel les une ou plusieurs unités monomères répétitives sont constituées d'atomes de C, N et H.

10. Capteur de l'une quelconque des revendications précédentes, dans lequel la couche polymère poreuse à effet de mèche est une couche de polyacrylonitrile poreux (PAN).

11. Capteur de l'une quelconque des revendications 1 à 10, dans lequel l'enzyme est au sein de la couche polymère poreuse à effet de mèche.

12. Capteur de l'une quelconque des revendications 1 à 11, dans lequel l'analyte est le glucose et l'enzyme est la glucose-oxydase.

13. Capteur de l'une quelconque des revendications 1 à 10, dans lequel la couche de grille polymère comprend un copolymère de fluoropolymère à base de tétrafluoroéthylène sulfoné, de préférence un copolymère d'acide tétrafluoroéthylène-perfluoro-3,6-dioxa-4-méthyl-7-octènesulfonique.

14. Capteur de l'une quelconque des revendications 1 à 13, dans lequel la couche semi-conductrice organique comprend un ou plusieurs polymères organiques choisis dans le groupe constitué par les polyacétylènes, les porphyrines, les phtalocyanines, les fullerènes, les polyparaphénylènes, les polyphénylènevinylènes, les polyfluorènes, les polythiophènes, les polypyrroles, les polypyridines, les polycarbazoles, les polypyridinevinylènes, les polyarylvinylènes, les poly(p-phénylméthylvinylènes), de préférence le poly-3-hexylthiophène.

15. Procédé de formation d'un capteur à base de transistor en couches minces organique (100, 200) selon l'une quelconque des revendications 1 à 14 destiné à détecter la présence d'un analyte dans un échantillon liquide aqueux, le procédé comprenant :
la fourniture d'une structure en couches comprenant au moins une couche semi-conductrice organique (122, 222) connectée à une électrode de source (104, 204) et à une électrode de drain (102, 202), et une couche de grille polymère (120, 220) ; et
la disposition d'une couche polymère poreuse à effet de mèche (108, 208) sur une surface de la couche de grille polymère ;
dans lequel la couche polymère poreuse à effet de mèche comprend, ou est traitée pour comprendre, une enzyme destinée à faciliter la génération d'une porteuse de charge à partir d'un analyte.

16. Procédé de la revendication 15,
dans lequel l'étape de disposition d'une couche polymère poreuse à effet de mèche sur la surface de la couche de grille polymère comprend la formation de la couche polymère poreuse à effet de mèche sur la surface de la couche de grille polymère par l'intermédiaire d'un procédé d'inversion de phase.

17. Procédé de la revendication 16,
dans lequel le procédé d'inversion de phase comprend :
la formation d'un film humide d'une solution polymère sur la surface d'un substrat, la solution polymère comprenant au moins un polymère dissous dans un solvant ; et
l'application d'un non-solvant sur le film humide, le non-solvant étant miscible avec le solvant, pour former une membrane polymère poreuse et pour dissoudre le solvant à partir de pores de la membrane polymère poreuse.
